# EUROPEAN PATENT SPECIFICATION

(11) **EP 3 334 488 B1**
(45) Date of publication and mention of the grant of the patent: **30.07.2025**
(21) Application number: 16835866.1
(22) Date of filing: 10.08.2016
(51) Int. Cl.: A61M 1/00, A61M 1/36, A61M 25/01, A61B 17/32

(54) **STEERABLE ASPIRATION CATHETER SYSTEM**
LENKBARES ABSAUGKATHETERSYSTEM
SYSTÈME DE CATHÉTER D'ASPIRATION ORIENTABLE

(30) Priority: 12.08.2015 US 201562204089 P
(43) Date of publication of application: 20.06.2018
(73) Proprietor: TDL Innovations, LLC, Princeton, NJ 08540 (US)
(72) Inventor: LAUB, Glenn, W., Princeton, NJ 08540 (US)
(74) Representative: Boult Wade Tennant LLP
(86) International application number: PCT/US2016/046431
(87) International publication number: WO 2017/027639

(56) References cited:
- WO-A1-96/30072
- DE-A1- 19 949 590
- US-A- 47 301
- US-A- 6 161 547
- US-A1- 2002 117 800
- US-A1- 2004 019 310
- US-A1- 2004 044 301
- US-A1- 2006 047 301
- US-A1- 2006 149 127
- US-A1- 2011 105 954
- US-A1- 2011 144 572
- US-A1- 2011 144 572
- US-A1- 2011 213 290
- US-A1- 2011 213 290
- US-A1- 2012 323 222
- US-A1- 2014 309 653
- US-A1- 2014 309 653
- US-B2- 8 205 917

## Description

### CROSS-REFERENCE TO RELATED APPLICATIONS

### FIELD OF THE INVENTION

The present invention, according to some embodiments, relates to a system for removing material from a patient's body. In some embodiments, the present invention relates to a system for aspirating, filtering and/or oxygenating a patient's blood. In some embodiments, the present invention relates to steerable catheter and a system incorporating the same. More particularly, the present invention in some embodiments relates to a steerable aspiration catheter system which is configured to remove material from the body of a patient, for example, unwanted material such as emboli, thrombi, tumors, or debris. In further embodiments, the present invention relates to an aspiration catheter system that is configured to return aspirated blood to the patient.

### BACKGROUND OF THE INVENTION

Thrombi within a person's vascular system can form as a result of any one of a number of causes, including disease, infections, trauma, surgery, stagnant blood, and foreign articles implanted in the vasculature. For example vascular disease, which annually affects a large number of individuals, often leads to the development of clots in the vascular system. These clots are usually comprised of an aggregated mixture of thrombus and fibrin and, if left untreated, may result in deep vein thrombosis, embolisms, or ischemia.

A thrombus present in a person's arterial system tends to move in the direction of blood flow from a large diameter artery to smaller diameter arteries and may continue to migrate until it becomes lodged against a vessel wall. In some instances, the thrombus partially or entirely blocks the flow of blood through the artery, thereby reducing or denying the supply of blood to tissue which is located downstream of the thrombus. Cutting off the flow of blood for an extended period of time may lead to damage or death of the tissue, possibly resulting in the loss of extremities in some cases. In other severe cases, a thrombus can migrate to the vessels of the brain and cause stroke and possibly death. Moreover, in a person's venous system, clots can migrate to the lungs and block the lungs main artery, resulting in a potentially fatal pulmonary embolism.
US2011/213290 describes a system for removing undesirable material from vessels and from chambers within the heart. The system includes a suction cannula for removing the undesirable material from a site of interest within a patient. A filtered device may be provided for capturing the undesirable material and removing it from the fluid flow. The system also includes a pump for generating the necessary suction force through the suction cannula to dislodge the undesirable material from the site of interest and for generating a sufficient driving force to direct the fluid flow downstream within the system. The system further includes a reinfusion cannula for reintroducing fluid removed from the site of interest back into a patient. US2006/0047301 A1 discloses an emboli removal system with oxygenated flow.

### SUMMARY OF THE INVENTION

The invention is defined by the appended claims. The present disclosure according to certain embodiments provides a system for removing thrombi and other unwanted material from the body of a patient, particularly from the patient's vasculature. As used herein, a patient may refer to a human patient, or in other embodiments, patient may also refer to non-human animals, for example, veterinary patients. In some embodiments, systems according to the present invention may be suited for thrombectomy and/or embolectomy procedures. Systems according to certain embodiments of the present invention may be used, for example, to remove clots from patients suffering from or at risk of pulmonary embolisms. In some embodiments, a system of the present invention is configured to aspirate thrombi and/or other unwanted material from the patient's vasculature. In some further embodiments, a system according to the present invention may be configured to add oxygen to and/or remove carbon dioxide from a patient's blood. In some embodiments, the system is further configured to return aspirated blood to the patient which, for example, allows for greater suction pressures and/or flow rates according to certain embodiments. In yet further embodiments, a system according to the present invention includes a steerable catheter to allow for directed aiming at the unwanted material.

In some embodiments, the system includes an aspiration catheter insertable into the patient having a distal end with a steerable tip configured to bend in one or more directions. In some embodiments, the system further includes a controller coupled to the aspiration catheter proximal to the distal end and operable by a user (e.g., surgeon) to manipulate and bend the steerable tip in the one or more directions. In some embodiments, the aspiration catheter includes one or more steering wires connected to the steerable tip, the controller configured to selectively apply tension on the one or more steering wires to bend the steerable tip in the one or more directions. In other embodiments, the aspiration catheter does not include a steering mechanism.

In further embodiments, the system includes a pump having an inlet port configured to be in fluid communication with the aspiration catheter. A filter device may be positioned between the aspiration catheter and the pump, the filter device configured to trap solid material (e.g., thrombi) received in the aspiration catheter from the body of the patient. The pump may also include a discharge port, the pump being configured to generate a negative pressure at the inlet port and a positive pressure at the discharge port during use. In yet other embodiments, the system includes a return catheter configured to be in fluid communication with the discharge port of the pump to return aspirated blood to the patient. The return catheter, in some embodiments, may or may not also include a steerable tip that is configured to bend in one or more directions in response to a controller. In some embodiments, the system may include an oxygenator configured to oxygenate the aspirated blood prior to returning the aspirated blood to the patient. In some such embodiments, the oxygenator is further configured to remove carbon dioxide from the aspirated blood.

In certain embodiments, the system includes at least one working port configured to allow insertion of one or more devices into or through the aspiration catheter. In some embodiments, the at least one working port is removably connected to the controller and/or aspiration catheter by a connector. The connector may be, for example, a quick connect fitting. In some embodiments, the at least one working port provides a fluid tight seal around the one or more devices when the one or more devices are inserted into the aspiration catheter. The one or more devices may include, for example, guidewires, stylets, balloon catheters, diagnostic catheters, baskets, optical fibers, thrombolysis tools, needles, cutters, biopsy devices, and other surgical implements known in the art. In some embodiments, the at least one working port includes a Tuohy Borst adaptor. In further embodiments, the system includes a plurality of working ports. Each of the plurality of working ports may be adapted to accept devices of different sizes. Moreover, each of the plurality of working ports may be removably connected to the controller and/or aspiration catheter via a separate connector.

In further embodiments, the present invention provides a stylet that is configured for use in positioning the aspiration catheter, the return catheter, and/or other catheters and cannulas. The stylet in some embodiments includes an elongate portion sized to fit within a lumen of the catheter or cannula. In some embodiments, the stylet further includes a steerable tip that is configured to bend in one or more directions. In some embodiments, the stylet includes a controller that is operable by a user to bend the steerable tip of the stylet in the one or more directions to help maneuver the stylet and catheter through a patient's vascular system during use.

### BRIEF DESCRIPTION OF THE DRAWINGS

The foregoing summary, as well as the following detailed description of the invention, will be better understood when read in conjunction with the appended drawings. For the purpose of illustrating the invention, there are shown in the drawings embodiments which are presently preferred. It should be understood, however, that the invention can be embodied in different forms and thus should not be construed as being limited to the embodiments set forth herein. Furthermore, unless noted otherwise, the appended drawings may not be drawn to scale.
FIG. 1A is a diagram illustrating a system for removing material from a patient's body in accordance with an embodiment of the present invention;
FIG. 1B is a diagram illustrating a steerable aspiration catheter system according to a further embodiment of the present invention;
FIG. 1C is a diagram illustrating a steerable aspiration catheter system according to a further embodiment of the present invention;
FIG. 1D is a diagram illustrating a steerable aspiration catheter system according to a further embodiment of the present invention;
FIG. 2 is a diagram illustrating a steerable aspiration catheter which may be used in the system shown in FIGS. 1A-1C in accordance with an embodiment of the present invention;
FIGS. 3A-3D are diagrams illustrating alternative distal tip configurations for a steerable aspiration catheter in accordance with embodiments of the present invention.
FIG. 4 is a diagram illustrating the steerable aspiration catheter of FIG. 2 receiving a guidewire and a stylet in accordance with an embodiment of the present invention;
FIG. 5 is a diagram illustrating multiple working ports in accordance with an embodiment of the present invention;
FIG. 6 is a diagram illustrating insertion of the steerable aspiration catheter into a vein in accordance with an embodiment of the present invention;
FIG. 7 is a diagram illustrating the positioning of the steerable aspiration catheter proximate a thrombus in accordance with an embodiment of the present invention;
FIG. 8 is a diagram illustrating a steerable stylet in accordance with an embodiment of the present invention; and
FIG. 9 is a diagram illustrating the steerable stylet of FIG. 7 positioned within a catheter in accordance with an embodiment of the present invention.

### DETAILED DESCRIPTION

The present subject matter will now be described more fully hereinafter with reference to the accompanying figures and examples, in which representative embodiments are shown. The present subject matter can, however, be embodied in different forms and should not be construed as limited to the embodiments set forth herein. Rather, these embodiments are provided to describe and enable one of skill in the art. The invention is defined by the appended claims.

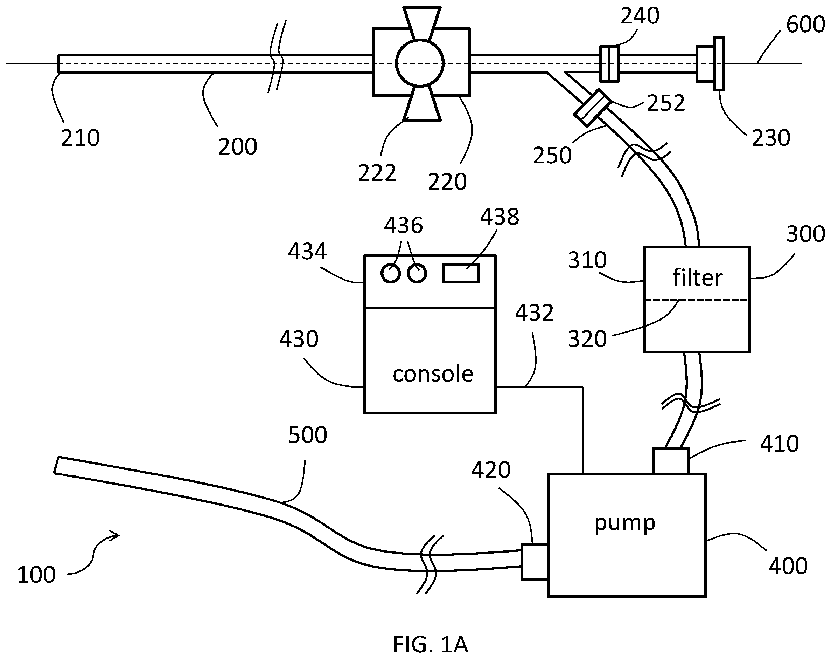

Referring to the drawings in detail, wherein like reference numerals indicate like elements throughout, there is shown in FIG. 1A a system, generally designated 100, for removing material from a patient's body in accordance with an exemplary embodiment of the present invention. In some embodiments, system 100 generally includes an aspiration catheter 200 configured to be inserted into the patient's body (e.g., into a vein or artery) and aspirate blood and other material (e.g., thrombi), a pump 400 for providing suction pressure to aspiration catheter 200, and a return catheter 500 for returning aspirated blood to the patient. More particularly, aspiration catheter 200 is arranged in fluid communication with inlet port 410 of pump 400 via fluid path 250 while return catheter 500 is in fluid communication with discharge port 420 of pump 400 such that, during use, blood from the patient is aspirated from a vein or artery through aspiration catheter 200 and fluid path 250 into inlet port 410 of pump 400 and expelled through discharge port 420 and return catheter 500 back to the patient. Return catheter 500 may be inserted into either a vein or artery of the pateint during use. Accordingly, in some embodiments, system 100 is configured to form a complete circuit with the patient's cardiovascular system. In some embodiments, only aspiration catheter 200 and return catheter 500 are inserted into the patient's body during aspiration while the other components of system 100 (e.g., fluid path 250, pump 400) remain outside the patient's body. In further embodiments, system 100 also includes a filter 300, which may be positioned anywhere in fluid communication between aspiration catheter 200 and return catheter 500. In some embodiments, filter 300 isdisposed between aspiration catheter 200 and pump 400 along fluid path 250 so as to separate any solid materials from the aspirated blood (e.g., thrombi, emboli, tumor tissue, etc.) before the aspirated blood enters pump 400. In other embodiments, a filter may be integrated into aspiration catheter 200 and/or pump 400.

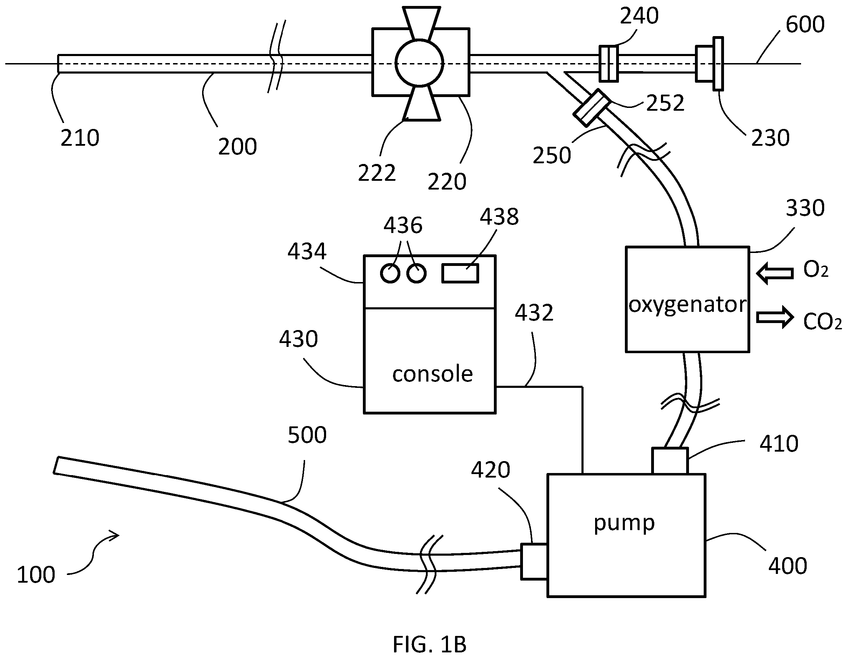

In some further embodiments, system 100 may include one or more other components which are configured to modify the aspirated blood before returning the blood to the patient. Referring to FIG. 1B, in some embodiments, for example, system 100 may include an oxygenator 330 which is configured to add oxygen to the aspirated blood before returning the blood to the patient. System 100 may be used, for example, to remove low-oxygen blood from the patient using aspiration catheter 200, add oxygen to the low-oxygen blood using oxygenator 330, and return the oxygenated blood to the patient via return catheter 500. Oxygenator 330 may also be configured to remove carbon dioxide from the aspirated blood in some embodiments. Oxygenator 330 may have any suitable configuration known in the art for adding oxygen to the blood. In one embodiment, for example, oxygenator 330 may include a membrane that is permeable to gas but impermeable to blood which is configured to allow oxygen to diffuse from an oxygen-containing gas (e.g., medical air) into the blood while carbon dioxide diffuses out of the blood into the gas for removal. In some such embodiments, system 100 may be particularly useful for extracorporeal membrane oxygenation (ECMO) procedures and/or heart assist procedures (e.g., right heart assist procedures). For example, in one embodiment, aspiration catheter 200 may be positioned to aspirate blood from a patient's vein (e.g., right common femoral vein) during use while return catheter 500 is inserted in either a vein (e.g., right internal jugular vein) or artery (e.g., right femoral artery) to return the aspirated blood to the patient after being modified by oxygenator 300. Oxygenator 330 may be positioned anywhere in fluid communication between aspiration catheter 200 and the end of return catheter 500, for example, along fluid path 250 between aspiration catheter 200 and inlet port 410 of pump 400 as illustrated. The blood oxygenator could alternatively be positioned between discharge port 420 of pump 400 and return catheter 500 in other embodiments. In yet other embodiments, oxygenator 330 and pump 400 could be integrated as a single device. System 100 shown in FIG. 1B may or may not include filter 300 discussed above in connection with FIG. 1A.

In further embodiments, system 100 may also include one or more infusion pumps (not shown) which are configured to introduce drugs, fluids, nutrients and/or other substances into the aspirated blood before the aspirated blood is returned to the patient via return catheter 500. Such infusion pumps may be connected to the system anywhere between aspiration catheter 200 and return catheter 500. In some embodiments, fluid path 250 or return catheter 500 may include one or more ports (not shown) which may be connected to an infusion pump and configured to receive material from the infusion pump for introduction into the aspirated blood. In yet other embodiments, system 100 may also include a temperature regulation device (e.g., heat exchanger) for modifying the temperature of the aspirated blood prior to returning the aspirated blood to the patient. In some embodiments, for example, blood may cool after it is aspirated from the patient's body. In some situations it may desirable to return the temperature of the aspirated blood towards normal body temperature (about 37 °C) before it returns to the patient. In some embodiments therefore, system 100 may include a heater or heat exchanger positioned along fluid path 250 and/or return catheter 500 that is configured to warm the aspirated blood.

Aspiration catheter 200 is preferably configured as an elongated tube which should be sufficiently flexible to allow for maneuverability through a patient's vasculature while also being stiff enough so as not to collapse under suction pressure from pump 400. In some embodiments, aspiration catheter 200 may be made from any material suitable for the manufacture of catheters. In some embodiments, aspiration catheter is made from a biocompatible polymer, for example, polyvinyl chloride, polyethylene, polypropylene, polyurethane, silicone, or combinations thereof. In some embodiments, aspiration catheter 200 may include reinforcing elements, for example, wires, coils, or ribs to prevent collapse during use.

In some embodiments, aspiration catheter 200 has a French size of at least 5 Fr. In some embodiments, aspiration catheter 200 has a French size of at least 6 Fr. In some embodiments, aspiration catheter 200 has a French size of at least 7 Fr. In some embodiments, aspiration catheter 200 has a French size of at least 8 Fr. In some embodiments, aspiration catheter 200 has a French size of at least 9 Fr. In some embodiments, aspiration catheter 200 has a French size of at least 10 Fr. In some embodiments, aspiration catheter 200 has a French size of at least 11 Fr. In some embodiments, aspiration catheter 200 has a French size of at least 12 Fr. In some embodiments, aspiration catheter 200 has a French size of at least 13 Fr. In some embodiments, aspiration catheter 200 has a French size of at least 14 Fr. In some embodiments, aspiration catheter 200 has a French size of at least 15 Fr. In some embodiments, aspiration catheter 200 has a French size of at least 16 Fr. In some embodiments, aspiration catheter 200 has a French size of at least 17 Fr. In some embodiments, aspiration catheter 200 has a French size of at least 18 Fr. In some embodiments, aspiration catheter 200 has a French size of at least 19 Fr. In some embodiments, aspiration catheter 200 has a French size of at least 20 Fr. In some embodiments, aspiration catheter 200 has a French size of 8 Fr to 12 Fr. In some embodiments, aspiration catheter 200 has a French size of 9 Fr to 11 Fr. In certain preferred embodiments, aspiration catheter has a French size of equal to or greater than 10 Fr to allow for aspiration of large thrombi and/or other solid materials from the patient.

In some embodiments, aspiration catheter 200 has a lumen diameter of at least 1 mm. In some embodiments, aspiration catheter 200 has a lumen diameter of at least 2 mm. In some embodiments, aspiration catheter 200 has a lumen diameter of at least 3 mm. In some embodiments, aspiration catheter 200 has a lumen diameter of at least 4 mm. In some embodiments, aspiration catheter 200 has a lumen diameter of at least 5 mm. In some embodiments, aspiration catheter 200 has a lumen diameter of 1 mm to 5 mm. In some embodiments, aspiration catheter 200 has a lumen diameter of 2 mm to 4 mm. In some embodiments, aspiration catheter 200 has a lumen diameter of equal to or greater than 3 mm.

Aspiration catheter 200, in certain preferred embodiments, is a steerable aspiration catheter. For example, in some embodiments, aspiration catheter 200 includes one or more steering wires which may extend along at least a portion of the length of aspiration catheter 200 to distal tip 210. In some embodiments, aspiration catheter 200 includes at least one pair of steering wires. In some embodiments, aspiration catheter 200 includes at least two pairs of steering wires. In some embodiments, aspiration catheter 200 includes at least three pairs of steering wires. In some embodiments, aspiration catheter 200 includes at least four pairs of steering wires. In some embodiments, aspiration catheter 200 includes at least five pairs of steering wires. In some embodiments, aspiration catheter 200 includes at least six pairs of steering wires. These steering wires may be positioned on the outside of aspiration catheter 200, inside the lumen of aspiration catheter 200, or within the walls of aspiration catheter 200. Each pair of steering wires may include diametrically opposed steering wires. By applying tension to selected steering wires, it is possible to cause distal tip 210 to bend in one or more directions. In some embodiments, being able to steer distal tip 210 of aspiration catheter 200 allows for better aiming of aspiration catheter towards the material to be removed from the patient (e.g., thrombi).

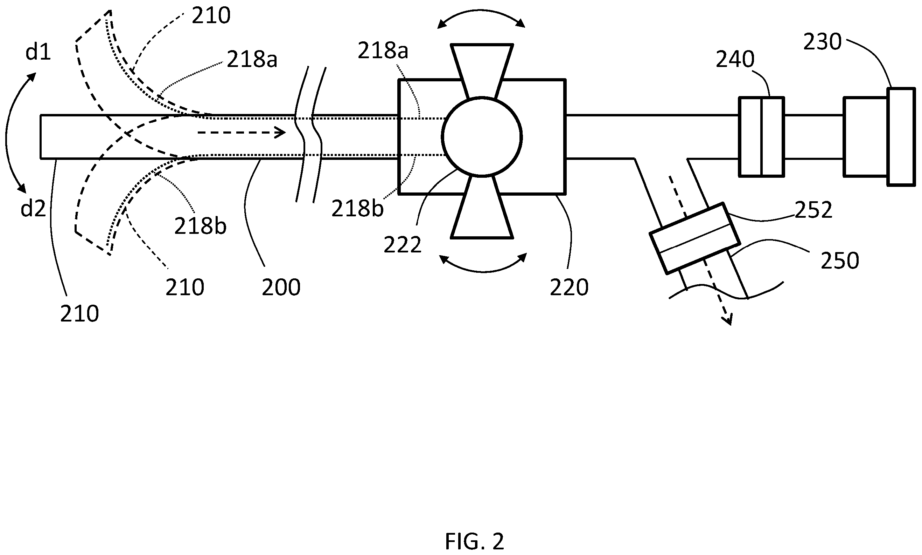

Referring now to FIG. 2, in some embodiments aspiration catheter 200 includes a distal tip 210 which is configured to bend in one or more directions in response to a controller 220 positioned proximally away from distal tip 210. Controller 220 may be configured to apply tension to one or more steering wires (e.g., 218a, 218b) which extend from controller 220 and are connected to distal tip 210. In some embodiments, steering wires 218a and 218b are positioned on the outside of aspiration catheter 200, inside the lumen of aspiration catheter 200, or within the walls of aspiration catheter 200. In some embodiments, steering wires 218a and 218b are diametrically opposed to each other. In some embodiments, controller 220 is positioned at a proximal end of aspiration catheter 200, opposite of distal tip 210. In some embodiments, controller 220 is mechanically coupled to aspiration catheter 200. In some embodiments, aspiration catheter 200 is partially received inside of controller 220. In some embodiments, controller 220 is configured as or includes a handle that is sized to be grasped by a user's hand.

Controller 220, in some embodiments, includes a dial or knob 222 which can be operated by a user (e.g., surgeon) to bend distal tip 210 in the one or more directions. For example, rotating dial or knob 222 clockwise may cause distal tip 210 to bend in a first direction d1 whereas rotating dial or knob 222 counterclockwise may cause distal tip 210 to bend in a second direction d2 which is opposite of first direction d1. For example, rotation of dial or knob 222 clockwise may pull steering wire 218a and/or push steering wire 218b causing distal tip 210 to bend in first direction d1. Rotation of dial or knob 222 counterclockwise may pull steering wire 218b and/or push steering wire 218a causing distal tip 210 to bend in second direction d2. Aspiration catheter 200 may include additional pairs of opposing steering wires to allow for bending in directions other than the ones illustrated for simplicity. It should be understood that controller 220 may also include additional dials or knobs to allow for bending of distal tip 210 in further directions not illustrated. It should also be appreciated that while controller 220 includes a dial or knob 222 in the illustrated embodiment for simplicity, other mechanisms such as levers, triggers, switches, thumbwheels, joysticks, buttons, slides, etc. could be used to operate controller 220 to steer distal tip 210. An example steering mechanism that may be adapted to the present system according to some embodiments is included in the DESTINO^{™} guiding sheath available from OSCOR^{®}.

In some embodiments, distal tip 210 is configured to bend from 0 degrees to about 10 degrees in one or more directions in response to operation of controller 220. In some embodiments, distal tip 210 is configured to bend from 0 degrees to about 15 degrees in one or more directions in response to operation of controller 220. In some embodiments, distal tip 210 is configured to bend from 0 degrees to about 20 degrees in one or more directions in response to operation of controller 220. In some embodiments, distal tip 210 is configured to bend from 0 degrees to about 25 degrees in one or more directions in response to operation of controller 220. In some embodiments, distal tip 210 is configured to bend from 0 degrees to about 30 degrees in one or more directions in response to operation of controller 220. In some embodiments, distal tip 210 is configured to bend from 0 degrees to about 40 degrees in one or more directions in response to operation of controller 220. In some embodiments, distal tip 210 is configured to bend from 0 degrees to about 45 degrees in one or more directions in response to operation of controller 220. In some embodiments, distal tip 210 is configured to bend from 0 degrees to about 50 degrees in one or more directions in response to operation of controller 220. In some embodiments, distal tip 210 is configured to bend from 0 degrees to about 60 degrees in one or more directions in response to operation of controller 220. In some embodiments, distal tip 210 is configured to bend from 0 degrees to about 65 degrees in one or more directions in response to operation of controller 220. In some embodiments, distal tip 210 is configured to bend from 0 degrees to about 70 degrees in one or more directions in response to operation of controller 220. In some embodiments, distal tip 210 is configured to bend from 0 degrees to about 75 degrees in one or more directions in response to operation of controller 220. In some embodiments, distal tip 210 is configured to bend from 0 degrees to about 80 degrees in one or more directions in response to operation of controller 220. In some embodiments, distal tip 210 is configured to bend from 0 degrees to about 85 degrees in one or more directions in response to operation of controller 220. In some embodiments, distal tip 210 is configured to bend from 0 degrees to about 90 degrees in one or more directions in response to operation of controller 220. In some embodiments, distal tip 210 is configured to bend from 0 degrees to about 100 degrees in one or more directions in response to operation of controller 220. In some embodiments, distal tip 210 is configured to bend from 0 degrees to about 135 degrees in one or more directions in response to operation of controller 220. In some embodiments, distal tip 210 is configured to bend from 0 degrees to about 180 degrees in one or more directions in response to operation of controller 220. In some embodiments, distal tip 210 is configured to bend no more than 90 degrees in any one direction. In some embodiments, distal tip 210 is configured to bend no more than 135 degrees in any one direction. In some embodiments, distal tip 210 is configured to bend no more than 180 degrees in any one direction.

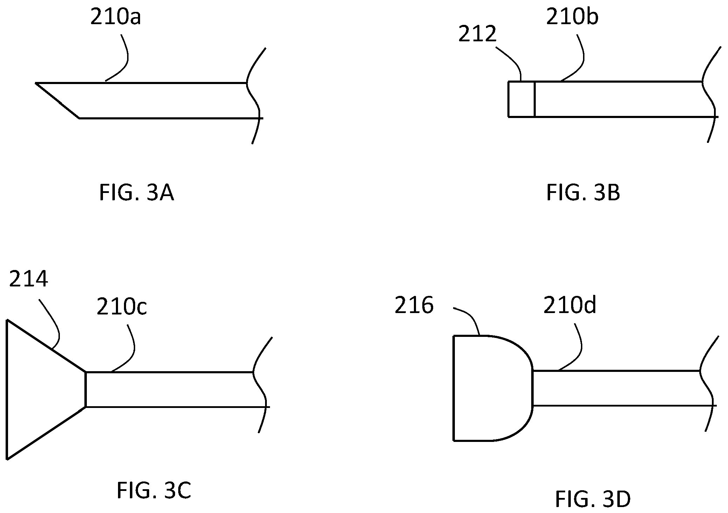

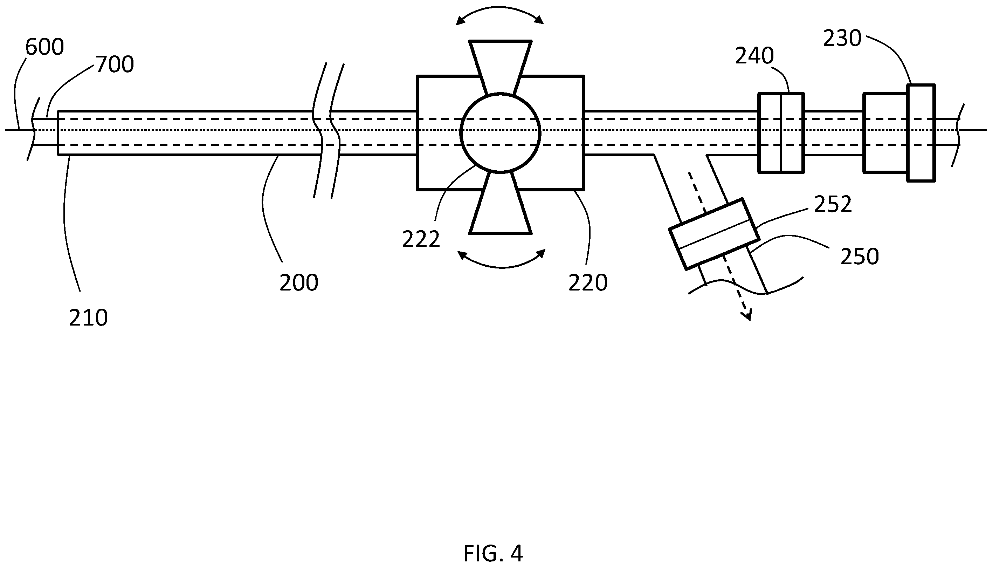

While FIG. 2 illustrates distal tip 210 as having a substantially flat end surface, distal tip 210 need not have such a configuration in other embodiments. Furthermore, distal tip 210 may be made from or include a different material than the rest of aspiration catheter 200. For example, distal tip 210 may be made from a more flexible or elastic material. FIGS. 3A-3D show other optional configurations that distal tip 210 may have. For example, FIG. 3A shows distal tip 210a as having a tapered or pointed end surface. FIG. 3B shows distal tip 210b which may be provided with a radiopaque marker 212 to allow for visualization of aspiration catheter 200 by radiographic imaging during use which permits the user (e.g., surgeon) to see the position of distal tip 210b within the patient's body. FIG. 3C illustrates an example distal tip 210c (not according to the claims) provided with a flared end or funnel 214 which may be deployed during use to help collect material during aspiration. Similarly, FIG. 3D shows an example distal tip 210d (not according to the claims) having a cup 216 which may be deployed during use to assist with material collection. It should be understood that funnel 214 and cup 216 need not be integral with aspiration catheter 200 and that they may be separate components which may be added to distal tip 210. According to the claimed invention, the distal end (210) of the aspiration catheter (200) comprises neither a funnel nor a cup. Other catheter end configurations and/or attachments may also be used with aspiration catheter 200. Preferably only end configurations which would not prevent or substantially interfere with the steering function of distal tip 210 are included.
Referring again to FIGS. 1A, 1B, and 2, extending proximally from controller 220 is a working port 230 according to some embodiments of the present invention. In some embodiments, working port 230 may be axially aligned with controller 220 and/or aspiration catheter 200. In some embodiments, working port 230 allows insertion of instruments into and/or through the lumen of aspiration catheter 200, such as, for example, guidewire 600 which may be used to assist with inserting aspiration catheter 200 into the patient's vasculature and directing aspiration catheter 200 to the location of the material to be removed. As further shown in FIG. 4, stylet 700 may also be inserted through aspiration catheter 200 via working port 230. Stylet 700 is an elongate tube which, in some embodiments provides a transition between the larger diameter aspiration catheter 200 and the smaller diameter guidewire 600. In further embodiments, stylet 700 helps provide rigidity to aspiration catheter 200 to help with insertion of aspiration catheter 200 through the patient's vasculature. In some embodiments, a plurality of coaxial stylets may be used. As depicted in the illustrated embodiments, guidewire 600 and stylet 700 have lengths which are greater than the length of aspiration catheter 200 and are configured to extend completely through working port 230, controller 220, and aspiration catheter 200 during use. Other devices not shown which may be inserted through aspiration catheter 200 via working port 230 include but are not limited to other wires, balloon catheters, diagnostic catheters, baskets, optical fibers, thrombolysis tools, needles, cutters, biopsy devices, and other surgical implements known in the art. In some embodiments, working port 230 may also be used to introduce additional fluids and/or medicaments into system 100.

In certain embodiments, working port 230 is configured to provide a fluid tight seal around stylet 700 or other device inserted through working port 230, for example, so as to prevent leakage of blood out of working port 230 during use. For example, working port 230 may include an o-ring seal that is sized to form a tight seal with stylet 700 or other tool inserted through working port 230. In some embodiments, working port 230 includes or is configured as a Tuohy-Borst adapter. In some embodiments, working port 230 may have an adjustable opening diameter to accommodate tools of different sizes. For example, working port 230 may be configured as a chuck, collet, adjustable collar, or other radial clamp. In yet further embodiments, working port 230 may include a valve to close working port 230 when not in use.

In some embodiments, working port 230 may be connected to controller 220 by a connector 240 which allows working port 230 to be detached and/or replaced. In some embodiments, connector 240 allows for working ports 230 which can accommodate different tools and devices to be exchanged. For example, a working port 230 which can accommodate tools of a particular size can be disconnected at connector 240 and exchanged for a different working port which can accommodate larger or smaller tools. In some embodiments, a plurality of different working ports 230, each of which being connectable to connector 240, may be provided as a kit for example. In some embodiments, connector 240 may be a quick connect fitting, threaded fitting, flanged fitting, or other tube fitting known in the art.

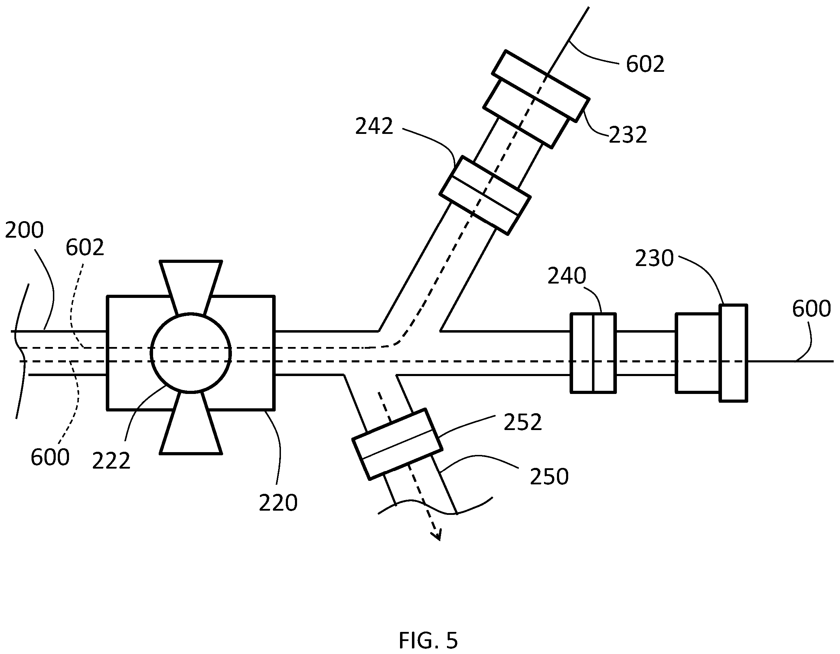

In yet further embodiments, system 100 may include more than one working port 230. In some embodiments, system 100 includes a plurality of working ports 230 so as to accommodate different tools and devices. FIG. 5 illustrates an embodiment having a second working port 232 in addition to working port 230, which may be connected via connector 242. Connector 242 may have a similar configuration as connector 240. As further shown in FIG. 5, working port 230 may receive guidewire 600 or other first tool while second working port 232 receives second guidewire 602 or other second tool. It should be understood that other embodiments may include additional working ports and that the total number of working ports is not limited to the illustrated embodiments. In some embodiments, system 100 includes at least one, at least two, at least three, or at least four working ports. Each of the one or more working ports may be the same or different in configuration. For example, working port 232 may be configured to have a different diameter than working port 230 such that working ports 230 and 232 can accommodate tools or devices of different sizes. Furthermore, each of the additional working ports can be connected via a separate connector which may be configured the same as connectors 240 and/or 242. By having connectors with the same or similar configurations, the different working ports may be interchanged with each other according to certain embodiments. In some embodiments, working ports 230 and 232 can be interchanged, for example, where working port 230 may be connected via connector 242 and second working port 232 can be connected via working port 240.

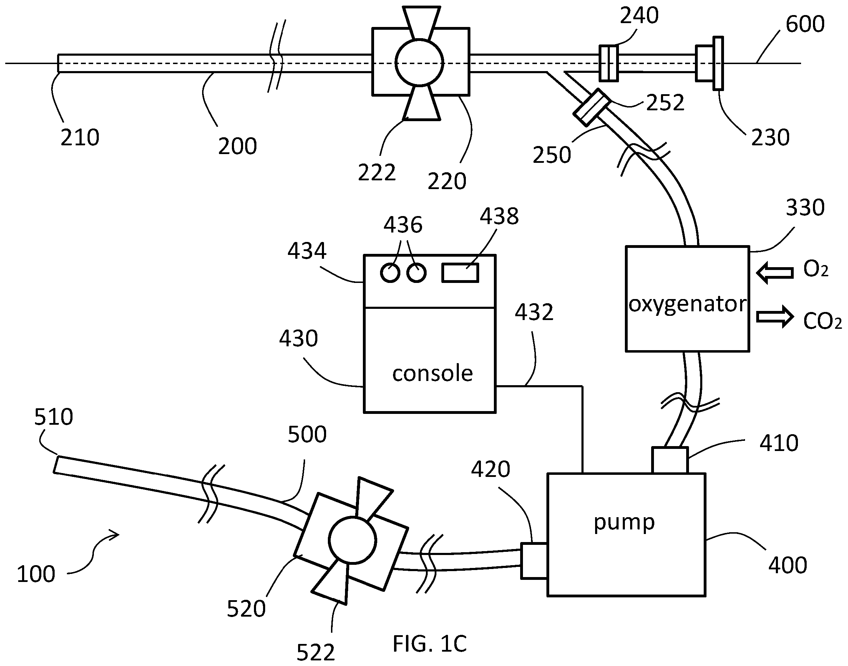

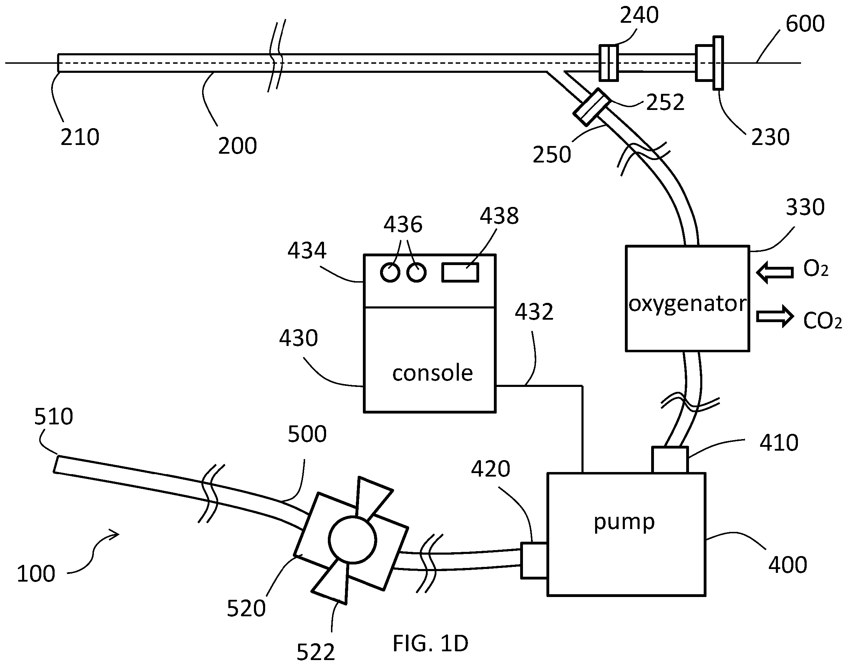

Referring again to FIGS. 1A and 1B, system 100 includes a fluid path 250 that places aspiration catheter 200 in fluid communication with inlet port 410 of pump 400. Fluid path 250 may be defined by an elongate tube which may be integral with or separable from aspiration catheter 200 according to certain embodiments. In some embodiments, for example, fluid path 250 may optionally connect with aspiration catheter 200 via a separate connector (e.g., connector 252) which may allow detachment of fluid path 250 from aspiration catheter 200. In some embodiments, the separate connector 252 may have a configuration similar to connector 240 and/or 242. In other embodiments, connector 252 may have a different configuration (e.g., different size) than connector 240 and/or 242. In some embodiments, fluid path 250 is continuous with aspiration catheter 200. While FIG. 1 depicts fluid path 250 as connecting to a location between controller 220 and connector 240, in other embodiments fluid path 250 may connect with aspiration catheter 200 at a position between distal tip 210 and controller 220. In yet other embodiments, fluid path 250 may connect at controller 220. Furthermore, it should be understood that in some alternative embodiments, the positions of fluid path 250 and working port 230 as shown in FIG. 1 may be switched such that fluid path 250 is axially aligned with controller 220 and/or aspiration catheter 200 while working port 230 branches in a different direction. For example, in some embodiments, fluid path 250 may connect at connector 240 while working port 230 connects at connector 252.

As shown in FIG. 1A, system 100 may be provided with a filter 300 according to some embodiments. In some embodiments, aspiration catheter 200 includes a filtering device. In some embodiments, filter 300 may be positioned anywhere downstream from distal tip 210 of aspiration catheter 200. In some embodiments, filter 300 may be positioned between aspiration catheter 200 and pump 400 along fluid path 250. In some embodiments, filter 300 is configured to trap solid material received through aspiration catheter 200 from the body of the patient during use. For example, filter 300 is configured to trap thrombi, emboli, tumor tissue, debris or other materials aspirated from the patient's body using system 100. Any suitable filtering apparatus known in the art may be used according to some embodiments. In some embodiments, filter 300 includes a housing 310 containing at least one separator 320 configured to separate the solid materials from the aspirated blood. Separator 320, in some embodiments, may be a screen, mesh, membrane, etc., that is configured to allow blood or other fluid to flow through while preventing passage of the solid materials. Blood which passes through separator 320 may then be suctioned through the remainder of fluid path 250 and into pump 400 through inlet port 410. Meanwhile, the solid materials collected within housing 310 of filter 300 can then be subsequently disposed of or retrieved for additional analysis. In other embodiments, pump 400 itself may include a filtering device. In yet other embodiments, filter 300 may be positioned downstream from pump 400 (e.g., along return catheter 500 or between pump 400 and return catheter 500). In some embodiments, filter 300 may be positioned anywhere between distal tip 210 of aspiration catheter 200 and the end of return catheter 500.

Pump 400, according to certain embodiments, is configured to create a suction force to drive system 100 during use. In preferred embodiments, pump 400 is a centrifugal pump. In other embodiments, pump 400 may be a rotary pump, peristaltic pump, roller pump, or other form of pump known in the art. In some embodiments, pump 400 may be controlled by a console 430 via communication pathway 432. Communication pathway 432 may be a hardwired electrical pathway, for example. In alternative embodiments, communication pathway 432 may be a wireless connection. In some embodiments, console 430 may be operated by the user (e.g., surgeon) to adjust the speed, pressure, or other attributes of pump 400 during use. In some embodiments, console 430 includes a control panel 434 which may receive input from the user to control pump 400. For example, control panel 434 may include one or more controls 436 (e.g., dials, touch screens, buttons, levers, etc.) for adjusting pump speed or other pump variable. Control panel 434 may also include other components such as, for example, one or more displays 438 (e.g., LCD display) that indicate pump speed, pressure or other values. In some embodiments, console 430 is a computer which may receive input from the user via a keyboard, mouse, etc. In some embodiments, console 430 and pump 400 may integrated as a single device.

Pump 400 is preferably configured to generate a negative (suction) pressure at inlet port 410 sufficient to cause aspiration of the patient's blood through aspiration catheter 200 during use. In some embodiments, pump 400 is capable of producing negative pressures from 0 mmHg to about -100 mmHg. In some embodiments, pump 400 is capable of producing negative pressures from 0 mmHg to about -150 mmHg. In some embodiments, pump 400 is capable of producing negative pressures from 0 mmHg to about -200 mmHg. In some embodiments, pump 400 is capable of producing negative pressures from 0 mmHg to about -250 mmHg. In some embodiments, pump 400 is capable of producing negative pressures from 0 mmHg to about -300 mmHg. In some embodiments, pump 400 is capable of producing negative pressures from 0 mmHg to about -350 mmHg. In some embodiments, pump 400 is capable of producing negative pressures from 0 mmHg to about -400 mmHg.

In some embodiments, pump 400 is configured to generate a blood flow rate of at least 100 mL/min through aspiration catheter 200. In some embodiments, pump 400 is configured to generate a blood flow rate of at least 200 mL/min through aspiration catheter 200. In some embodiments, pump 400 is configured to generate a blood flow rate of at least 300 mL/min through aspiration catheter 200. In some embodiments, pump 400 is configured to generate a blood flow rate of at least 400 mL/min through aspiration catheter 200. In some embodiments, pump 400 is configured to generate a blood flow rate of at least 500 mL/min through aspiration catheter 200. In some embodiments, pump 400 is configured to generate a blood flow rate of at least 600 mL/min through aspiration catheter 200. In some embodiments, pump 400 is configured to generate a blood flow rate of at least 700 mL/min through aspiration catheter 200. In some embodiments, pump 400 is configured to generate a blood flow rate of at least 800 mL/min through aspiration catheter 200. In some embodiments, pump 400 is configured to generate a blood flow rate of at least 900 mL/min through aspiration catheter 200. In some embodiments, pump 400 is configured to generate a blood flow rate of at least 1000 mL/min through aspiration catheter 200. In some embodiments, pump 400 is configured to generate a blood flow rate of at least 1100 mL/min through aspiration catheter 200. In some embodiments, pump 400 is configured to generate a blood flow rate of at least 1200 mL/min through aspiration catheter 200. In some embodiments, pump 400 is configured to generate a blood flow rate of at least 1300 mL/min through aspiration catheter 200. In some embodiments, pump 400 is configured to generate a blood flow rate of at least 1400 mL/min through aspiration catheter 200. In some embodiments, pump 400 is configured to generate a blood flow rate of at least 1500 mL/min through aspiration catheter 200. In some embodiments, pump 400 is configured to generate a blood flow rate of at least 1600 mL/min through aspiration catheter 200. In some embodiments, pump 400 is configured to generate a blood flow rate of at least 1700 mL/min through aspiration catheter 200. In some embodiments, pump 400 is configured to generate a blood flow rate of at least 1800 mL/min through aspiration catheter 200. In some embodiments, pump 400 is configured to generate a blood flow rate of at least 1900 mL/min through aspiration catheter 200. In some embodiments, pump 400 is configured to generate a blood flow rate of at least 2000 mL/min through aspiration catheter 200. In some embodiments, pump 400 is configured to generate a blood flow rate of at least 3000 mL/min through aspiration catheter 200. In some embodiments, pump 400 is configured to generate a blood flow rate of at least 4000 mL/min through aspiration catheter 200. In some embodiments, pump 400 is configured to generate a blood flow rate of at least 5000 mL/min through aspiration catheter 200.

In some embodiments, pump 400 is configured to generate a blood flow rate of about 100 mL/min to about 6000 mL/min through aspiration catheter 200. In some embodiments, pump 400 is configured to generate a blood flow rate of about 1000 mL/min to about 5000 mL/min through aspiration catheter 200. In some embodiments, pump 400 is configured to generate a blood flow rate of about 1500 mL/min to about 4500 mL/min through aspiration catheter 200. In some embodiments, pump 400 is configured to generate a blood flow rate of about 2000 mL/min to about 4000 mL/min through aspiration catheter 200. In some embodiments, pump 400 is configured such that the generated flow rate may be ramped up from 0 mL/min to the desired flow rate during use (e.g., via console 430).

In further embodiments, pump 400 includes a discharge port 420 separate from inlet port 410. Pump 400 is configured to generate a positive pressure at discharge port 420 such that the aspirated blood received through inlet port 410 is expelled through discharge port 420 during use. The aspirated blood or other fluids are typically expelled through discharge port 420 at about the same flow rate as the flow rate into pump 400 through inlet port 410. As shown in FIG. 1, system 100 may include a return catheter 500 in fluid communication with discharge port 420. In some embodiments, return catheter 500 is configured to carry aspirated blood expelled from discharge port 420 back to the patient. By returning the aspirated blood back to the patient, embodiments of the present system 100 allows for aspiration while minimizing the blood loss of the patient. In certain embodiments, reinfusing the patient's blood continuously during aspiration allows for greater suction pressure and/or flow rates (e.g., 2-4 L/min) which can assist in dislodging and removing larger clots and/or tumors than would otherwise be possible. Without returning the blood back to the patient, such high flow rates could quickly result in exsanguination of the patient. Return catheter 500 in some embodiments is an elongate, flexible tube which is particularly configured to be inserted into the patient's vasculature. In some embodiments, return catheter 500 may be inserted into the patient's peripheral venous system. In other embodiments, return catheter 500 may be inserted into the patient's arterial system. Return catheter 500 may be inserted into the patient's vascular system with the aid of a guidewire and/or stylet as described herein in connection with aspiration catheter 200. For example, a guidewire and/or stylet having a configuration as guidewire 600 and/or stylet 700 may be used for positioning return catheter 500 in the desired location within the patient's vascular system. The guidewire and/or stylet may be removed from return catheter 500 after proper positioning of return catheter 500. In some embodiments, return catheter 500 is connected to pump 420 after it is positioned within the patient's body. As with aspiration catheter 200, return catheter 500 may be similarly made from a biocompatible polymer. In some embodiments, return catheter 500 may also include a radiopaque marker (not shown) to assist with visualization by radiographic imaging. In further embodiments, return catheter 500 may have about the same diameter as aspiration catheter 200. In further embodiments, a second filter (not shown) may be positioned along return catheter 500 or at discharge port 420 such that aspirated blood exiting discharge port 420 of pump 400 may be further filtered of debris or other undesired materials prior to being returned to the patient. In some embodiments, return catheter 500 does not include a direct steering mechanism. In alternative embodiments, return catheter 500 may be a steerable catheter. For example, in some embodiments, return catheter 500 may include a steerable tip that is operable using a controller similar to the configuration as described for aspiration catheter 200. In some embodiments, for example as shown in FIGS. 1C and 1D, return catheter 500 may include a tip 510 which is configured to be bend in one or more directions in response to a controller 520 spaced away from tip 510. Controller 520 may be positioned between tip 510 and discharge port 420 of pump 400 during use according to some embodiments. Tip 510 and controller 520 may have the same configuration as described for distal tip 210 and controller 220. For instance, in some embodiments, controller 520 may mechanically coupled to return catheter 500 and may be configured to apply tension to one or more steering wires (not shown) which extend from controller 520 and are connected to tip 510 in order to steer tip 510 in one or more directions. In some embodiments, controller 520 includes a dial or knob 522 which can be operated by a user (e.g., surgeon) to bend tip 510 in the one or more directions. Tip 510 may be configured to bend to the same degree as described above for distal tip 210. In some embodiments, being able to steer tip 510 of return catheter 500 may help precisely position return catheter 500 in the patient's vasculature. This configuration may be particularly useful, for example, in extracorporeal membrane oxygenation (ECMO) procedures and/or heart assist procedures (e.g., right heart assist procedures) according to some embodiments. As shown in FIG. 1C, each of aspiration catheter 200 and return catheter 500 in system 100 may be steerable. In certain alternative embodiments, as shown in FIG. 1D, return catheter 500 is steerable while aspiration catheter 200 is a non-steerable catheter. As used in embodiments described herein, a non-steerable catheter refers to a catheter that does not incorporate a direct steering mechanism. While system 100 shown in FIGS. 1C and 1D includes oxygenator 330, in other embodiments oxygenator 330 need not be included. Moreover, it should be understood that the other components described herein in connection with system 100 shown in FIGS. 1A and 1B may also be included in the embodiments of system 100 shown in FIGS. 1C and 1D. For example, system 100 shown in FIGS. 1C and 1D may include a filter which can be configured and positioned in the same manner as filter 300 described herein with reference to FIG. 1A.

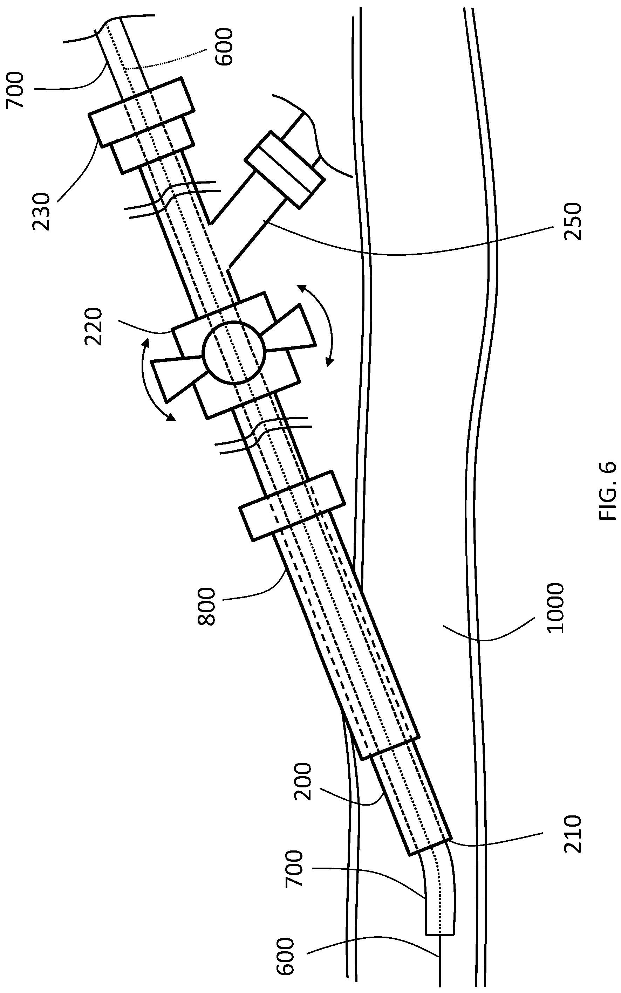

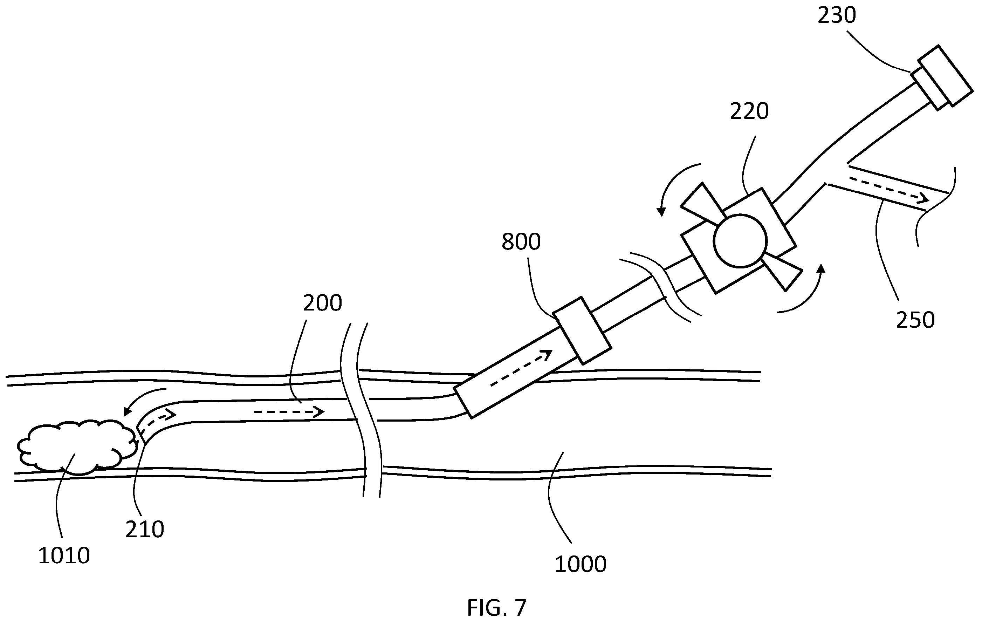

FIG. 6 provides an illustration of aspiration catheter 200 inserted into vein 1000 according to one embodiment. As shown in this embodiment, aspiration catheter 200 is inserted into vein 1000 over guidewire 600 and stylet 700 in a coaxial arrangement. Guidewire 600 and stylet 700 are sized such that their ends extend beyond the distal tip 210 of aspiration catheter 200 and beyond working port 230. Moreover catheter 200 may be inserted through a tubular sheath 800 which is configured to provide a port into vein 1000. Components such as controller 220, working port 230, fluid path 250 remain outside the patient's body. As described herein, guidewire 600 and stylet 700 may be used to maneuver aspiration catheter 200 through vein 1000 to the desired location within the patient's vascular system. After aspiration catheter 200 has been positioned into the desired location inside the patient's vasculature, guidewire 600 and stylet 700 can be withdrawn from aspiration catheter 200 through working port 230 according to some embodiments while aspiration catheter 200 remains in place. Following withdrawal of guidewire 600 and stylet 700, working port 230 may be sealed so as to prevent leakage of blood through working port 230. Activation of pump 400 (FIGS. 1A, 1B) will then cause aspiration catheter 200 to aspirate blood and/or other materials from the patient which will flow through distal tip 210 into the lumen of aspiration catheter 200 and through fluid path 250 to the other components of system 100 described above. Console 430 may be used to regulate the pressure and/or speed of pump 400 as described above. Where aspiration catheter 200 is a steerable aspiration catheter, as shown for example in FIG. 7, controller 220 may be used to turn and aim distal tip 210 toward thrombus 1010 to facilitate suction and removal of thrombus 1010 from the patient through aspiration catheter 200 along the pathway depicted by dashed arrows. Precise positioning of distal tip 210 can be verified, for example, using radiographic imaging according to some embodiments. Referring again to system 100 shown in FIG. 1A, the aspirated blood may be filtered through filter 300 to separate out the thrombus and/or other materials from the blood according to some embodiments, and the blood then returned to the patient via return catheter 500 which may be inserted into the patient's venous or arterial system. The aspirated blood may be modified by other devices, for example, oxygenator 330 of FIG. 1B prior to being returned to the patient.

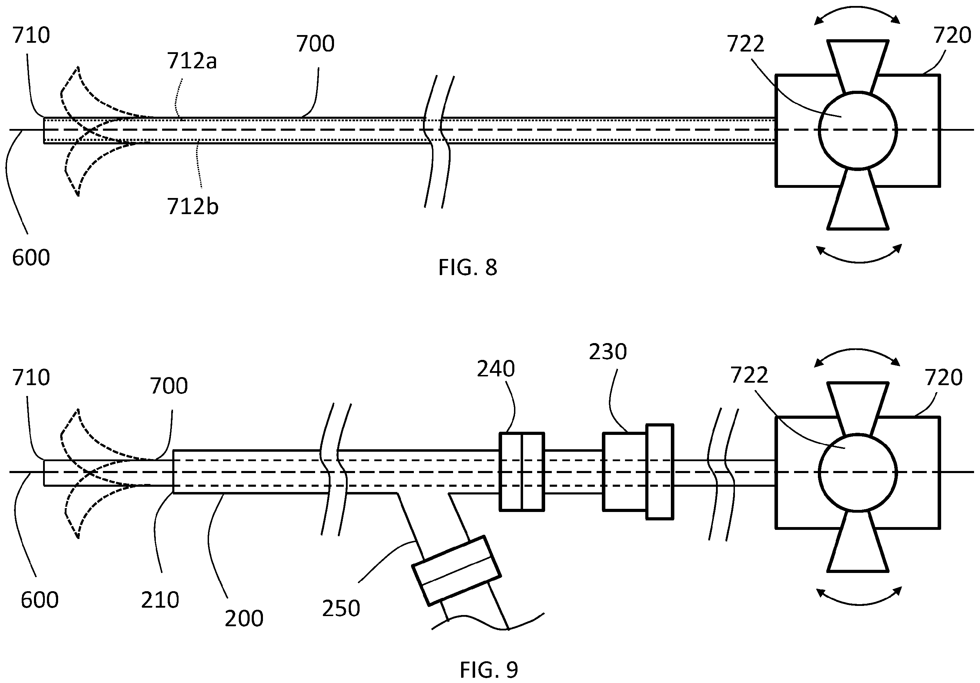

Referring now to FIGS. 8 and 9, there is shown an example stylet 700 according to an embodiment of the present invention that may be utilized in the positioning of aspiration catheter 200 within the patient's vascular system. In some embodiments, stylet 700 may be utilized in the positioning of return catheter 500 within the patient's vascular system. In some embodiments, stylet 700 includes an elongate, flexible tube having an outer diameter that is sized to fit within aspiration catheter 200 and an inner (lumen) diameter sized to allow passage of guidewire 600 there through. In some embodiments, stylet 700 has an inside diameter that is sized to form a tight fit around guidewire 600. In some embodiments, stylet 700 is not configured to permit passage of liquid through its lumen when guidewire 600 is received there through. The outer diameter of stylet 700 should be selected to fit within the lumen of the catheter that stylet 700 is being used to position. In some embodiments, the outer diameter of stylet 700 is less than 5 mm, less than 4 mm, less than 3 mm, less than 2 mm, or less than 1 mm. In further embodiments, stylet 700 has a length that is longer than the length of the catheter that stylet 700 is being used to position.

In some embodiments, stylet 700 may be a steerable stylet having a distal tip 710 which is configured to bend in one or more directions in response to a controller 720 positioned proximally away from distal tip 710. Controller 720 may be configured to apply tension to one or more steering wires 712a,712b which extend from controller 720 and are connected to distal tip 710. By applying tension to selected steering wires, it is possible to cause distal tip 710 to bend in one or more directions to help steer stylet during insertion into the patient's vasculature. In some embodiments, steering wires 712a, 712b may be positioned on the outside of stylet 700, inside the lumen of stylet 700, or within the tube walls of stylet 700. In some embodiments, stylet 700 includes one or more pairs of steering wires which are diametrically opposed to each other.

Controller 720, in some embodiments, includes a dial or knob 722 which can be operated by a user (e.g., surgeon) to bend distal tip 710 in the one or more directions. For example, rotating dial or knob 722 clockwise may cause distal tip 710 to bend in a first direction whereas rotating dial or knob 722 counterclockwise may cause distal tip 710 to bend in a second direction which is opposite of first direction d1. For example, rotation of dial or knob 722 clockwise may pull steering wire 718a and/or push steering wire 718b causing distal tip 710 to bend in first direction. Rotation of dial or knob 722 counterclockwise may pull steering wire 718b and/or push steering wire 718a causing distal tip 710 to bend in second direction. Stylet 700 may include additional pairs of opposing steering wires to allow for bending in further directions. It should be understood that controller 720 may also include additional dials or knobs to allow for bending of distal tip 710 in these further directions. It should also be appreciated that while controller 720 includes a dial or knob 722 in the illustrated embodiment for simplicity, other mechanisms such as levers, triggers, switches, thumbwheels, joysticks, buttons, slides, etc. could be used to operate controller 720 to steer distal tip 710. Controller 720 may be configured to have guidewire 600 extend through it in some embodiments. An example steering mechanism that may be adapted to the present system according to some embodiments is included in the DESTINO^{™} guiding sheath available from OSCOR^{®}. In some embodiments, stylet 700 does not include any working ports.

In some embodiments, distal tip 710 is configured to bend from 0 degrees to about 10 degrees in one or more directions in response to operation of controller 720. In some embodiments, distal tip 710 is configured to bend from 0 degrees to about 15 degrees in one or more directions in response to operation of controller 720. In some embodiments, distal tip 710 is configured to bend from 0 degrees to about 20 degrees in one or more directions in response to operation of controller 720. In some embodiments, distal tip 710 is configured to bend from 0 degrees to about 25 degrees in one or more directions in response to operation of controller 720. In some embodiments, distal tip 710 is configured to bend from 0 degrees to about 30 degrees in one or more directions in response to operation of controller 720. In some embodiments, distal tip 710 is configured to bend from 0 degrees to about 40 degrees in one or more directions in response to operation of controller 720. In some embodiments, distal tip 710 is configured to bend from 0 degrees to about 45 degrees in one or more directions in response to operation of controller 720. In some embodiments, distal tip 710 is configured to bend from 0 degrees to about 50 degrees in one or more directions in response to operation of controller 220. In some embodiments, distal tip 710 is configured to bend from 0 degrees to about 60 degrees in one or more directions in response to operation of controller 720. In some embodiments, distal tip 710 is configured to bend from 0 degrees to about 65 degrees in one or more directions in response to operation of controller 720. In some embodiments, distal tip 710 is configured to bend from 0 degrees to about 70 degrees in one or more directions in response to operation of controller 720. In some embodiments, distal tip 710 is configured to bend from 0 degrees to about 75 degrees in one or more directions in response to operation of controller 720. In some embodiments, distal tip 710 is configured to bend from 0 degrees to about 80 degrees in one or more directions in response to operation of controller 720. In some embodiments, distal tip 710 is configured to bend from 0 degrees to about 85 degrees in one or more directions in response to operation of controller 720. In some embodiments, distal tip 710 is configured to bend from 0 degrees to about 90 degrees in one or more directions in response to operation of controller 720. In some embodiments, distal tip 710 is configured to bend from 0 degrees to about 100 degrees in one or more directions in response to operation of controller 720. In some embodiments, distal tip 710 is configured to bend from 0 degrees to about 135 degrees in one or more directions in response to operation of controller 720. In some embodiments, distal tip 710 is configured to bend from 0 degrees to about 180 degrees in one or more directions in response to operation of controller 720. In some embodiments, distal tip 710 is configured to bend no more than 90 degrees in any one direction. In some embodiments, distal tip 710 is configured to bend no more than 135 degrees in any one direction. In some embodiments, distal tip 710 is configured to bend no more than 180 degrees in any one direction.

FIG. 9 shows stylet 700 positioned through working port 230 and aspiration catheter 200 according to one embodiment. Stylet 700 may have a length that is longer than a length of aspiration catheter 200 such that distal tip 710 of stylet 700 extends beyond distal tip 210 of aspiration catheter 210 during use. Stylet 700 may also extend beyond working port 230. As discussed above, in some embodiments, stylet 700 may be configured to provide a transition between the larger diameter aspiration catheter 200 and the smaller diameter guidewire 600. In further embodiments, stylet 700 helps provide rigidity to aspiration catheter 200 to help with insertion of aspiration catheter 200 through the patient's vasculature. In some embodiments, having a distal tip 710 that can be steered using controller 720 helps to maneuver and position stylet 700 and aspiration catheter 200 into the desired position within the patient's vasculature. Once catheter 200 is positioned, stylet 700 can be withdrawn from aspiration catheter 200 (e.g., through working port 230) while aspiration catheter 200 remains in place as described above. It should be understood that controller 720 would remain outside of the patient's body during use. In some embodiments, stylet 700 may be maneuvered together with aspiration catheter 200 coaxially positioned around stylet 700 through the patient's vasculature. In some embodiments, stylet 700 is first inserted into the patient's vasculature and is navigated through the patient's vasculature until distal tip 710 reaches a desired location within a vessel. Aspiration catheter 200 may then be slid coaxially over stylet 700 until distal tip 210 of aspiration catheter 200 reaches the desired location, and stylet 700 can then be withdrawn from aspiration catheter 200 (e.g., through working port 230) while aspiration catheter 200 remains in place within the vessel. In some embodiments, return catheter 500 can be similarly positioned within a patient's vasculature using a steerable stylet. In some embodiments, a system according to the present invention includes a separate stylet 700 for each of aspiration catheter 200 and return catheter 500. This may be appropriate, for example, in embodiments where aspiration catheter 200 and return catheter 500 have different lumen diameters. In certain other embodiments, aspiration catheter 200 and return catheter may be used with the same stylet 700.

It should be understood that stylet 700, in some embodiments, may be used for positioning steerable or non-steerable catheters. For example, in certain further embodiments of the present invention, both aspiration catheter 200 and return catheter 500 are non-steerable catheters and do not include direct steering mechanisms. In some such embodiments, one or both of aspiration catheter 200 and return catheter 500 may be positioned within a desired location within a patient's blood vessel by way of a steerable stylet 700, which is then removed from aspiration catheter 200 and/or return catheter 500. In other embodiments, only one of aspiration catheter 200 and return catheter is steerable and includes a direct steering mechanism. A steerable stylet 700 may be used for positioning the steerable catheter or the non-steerable catheter.

It should also be appreciated that other stylets that are known in the art may be alternatively used for positioning aspiration catheter 200 and/or return catheter 500, for example, non-steerable stylets. It should also be appreciated that stylet 700 described herein is not necessarily limited to use with the aspiration system of the present invention. For example, stylet 700 may also be used for positioning catheters other than aspiration catheter 200 or return catheter 500. In further embodiments, stylet 700 may be configured for positioning catheters and cannulas that are configured for use in other medical procedures. For example, in other embodiments, stylet 700 with steerable distal tip 710 may be particularly useful for maneuvering a cannula through a patient's pulmonary artery for positioning a right heart assist device. In one such embodiment, stylet 700 would be positioned within the lumen of the cannula and the assembly steered into position within the pulmonary artery. Once in the desired location, stylet 700 would be withdrawn from the cannula while leaving the cannula in place. Stylet 700 could likewise be adapted for use in positioning other existing catheters and cannulas known in the art. In yet further embodiments, a steerable stylet 700 could be used for positioning a catheter or cannula without additional guidewire 600. In some such embodiments, stylet 700 need not be hollow or include a lumen.

It is to be understood that at least some of the figures and descriptions of the invention have been simplified to focus on elements that are relevant for a clear understanding of the invention, while eliminating, for purposes of clarity, other elements that those of ordinary skill in the art will appreciate may also comprise a portion of the invention. However, because such elements are well known in the art, and because they do not necessarily facilitate a better understanding of the invention, a further description of such elements is not provided herein.

It will be further appreciated by those skilled in the art that changes could be made to the exemplary embodiments shown and described above without departing from the invention as defined by the appended claims. Moreover, the scope of the present application is not intended to be limited to the particular embodiments of the process, machine, manufacture, and composition of matter, means, methods and steps described in the specification. As one of ordinary skill in the art will readily appreciate from the disclosure herein, processes, machines, manufacture, composition of matter, means, methods, or steps, that perform substantially the same function or achieve substantially the same result as the corresponding embodiments described herein may be utilized according to the present disclosure. Moreover, unless specifically set forth herein, the terms "a," "an," and "the" are not limited to one element but instead should be read as meaning "at least one."

## Claims

1. A system (100) for removing unwanted material from the body of a patient, the system comprising: an aspiration catheter (200) insertable into the patient comprising a distal end (210) having a steerable tip configured to bend in one or more directions, wherein the distal end (210) of the aspiration catheter (200) comprises neither a funnel nor a cup; a controller (220) mechanically coupled to the aspiration catheter (200) and operable by a user to bend the steerable tip in the one or more directions; a pump (400) comprising an inlet port (410) and a discharge port (420), the inlet port (410) being in fluid communication with the aspiration catheter (200), the pump (400) configured to generate a negative pressure at the inlet port (410) and a positive pressure at the discharge port (420) during use; and a return catheter (500) in fluid communication with the discharge port (420) of the pump (400) and configured to be inserted into the patient, wherein the unwanted material comprises one or more of emboli, thrombi, tumors, or debris.

2. The system according to claim 1,
wherein the aspiration catheter (200) has a size equal to or greater than 10 Fr; or
wherein the aspiration catheter (200) has a lumen diameter of at least 3 mm; or
wherein the negative pressure at the inlet port (410) is between 0 mmHg to about -150 mmHg.

3. The system according to claim 1,
wherein the pump (400) is configured to generate a flow rate of at least 100 mL/min through the aspiration catheter (200); or
wherein the pump is configured to generate a flow rate of about 2 L/min to about 4 L/min through the aspiration catheter.

4. The system according to claim 1, further comprising at least one working port (230) configured to allow insertion of one or more devices into the aspiration catheter (200).

5. The system according to claim 4,
wherein the one or more devices includes a stylet (700); or
wherein the one or more devices includes a guidewire (600).

6. The system according to claim 4,
wherein the at least one working port (230) includes a Tuohy Borst adaptor; or
wherein the at least one working port (230) provides a fluid tight seal around the one or more devices when the one or more devices are inserted into the aspiration catheter (200).

7. The system according to claim 4, wherein the at least one working port (230) is removably connected to the controller (220) by a connector (240); and optionally
wherein the connector is a quick connect fitting.

8. The system according to claim 4, wherein the at least one working port (230) comprises at least two working ports (230, 232) having different sizes.

9. The system according to claim 1, wherein the aspiration catheter (200) includes one or more steering wires (218a, 218b) connected to the steerable tip, the controller (220) configured to selectively apply tension on the one or more steering wires (218a, 218b) to bend the steerable tip in the one or more directions.

10. The system according to claim 1, wherein the steerable tip is configured to bend at an angle of about 0 to about 90 degrees.

11. The system according to claim 1, further comprising a filter device (300) positioned in fluid communication between the aspiration catheter (200) and the return catheter (500), the filter device (300) configured to trap solid material received in the aspiration catheter (200) from the body of the patient.

12. The system according to claim 1, further comprising an oxygenator (330) configured to add oxygen to and/or remove carbon dioxide from blood from the patient, the oxygenator (330) being positioned in fluid communication between the aspiration catheter (200) and the return catheter (500).

13. The system according to claim 1, further comprising a stylet (700) sized and configured to be received within a lumen of the aspiration catheter (200) or the return catheter (500); and optionally
wherein the stylet (700) comprises a steerable tip and a controller (220) operable by a user to bend the steerable tip of the stylet in one or more directions.

14. The system according to claim 1, wherein the system includes a second controller (520) and the return catheter (500) includes a steerable tip (510) configured to bend in one or more directions in response to operation of the second controller (520).

## Patentansprüche

1. System (100) zum Entfernen von unerwünschtem Material aus dem Körper eines Patienten, wobei das System umfasst: einen Saugkatheter (200), der in den Patienten einführbar ist und ein distales Ende (210) mit einer steuerbaren Spitze umfasst, die konfiguriert ist, um sich in eine oder mehrere Richtungen zu biegen, wobei das distale Ende (210) des Saugkatheters (200) weder einen Trichter noch eine Glocke umfasst; eine Steuereinheit (220), die mechanisch mit dem Saugkatheter (200) gekoppelt ist und von einem Benutzer betätigbar ist, um die steuerbare Spitze in die eine oder mehrere Richtungen zu biegen; eine Pumpe (400), die einen Einlassanschluss (410) und einen Auslassanschluss (420) umfasst, wobei der Einlassanschluss (410) in Fluidverbindung mit dem Saugkatheter (200) steht, wobei die Pumpe (400) konfiguriert ist, um bei Gebrauch einen Unterdruck am Einlassanschluss (410) und einen Überdruck am Auslassanschluss (420) zu erzeugen; und einen Rückführkatheter (500), der in Fluidverbindung mit dem Auslassanschluss (420) der Pumpe (400) steht und konfiguriert ist, um in den Patienten eingeführt zu werden, wobei das unerwünschte Material Embolien, Thromben, Tumoren und/oder Ablagerungen umfasst.

2. System nach Anspruch 1,
bei dem der Saugkatheter (200) eine Größe von mindestens 10 Fr aufweist; oder
bei dem der Saugkatheter (200) einen Lumendurchmesser von mindestens 3 mm aufweist; oder
bei dem der Unterdruck an der Einlassöffnung (410) zwischen 0 mmHg und etwa -150 mmHg liegt.

3. System nach Anspruch 1,
bei dem die Pumpe (400) konfiguriert ist, um eine Durchflussrate von mindestens 100 ml/min durch den Saugkatheter (200) zu erzeugen; oder
bei dem die Pumpe konfiguriert ist, um eine Durchflussrate von etwa 2 l/min bis etwa 4 l/min durch den Saugkatheter zu erzeugen.

4. System nach Anspruch 1, das ferner mindestens einen Arbeitsanschluss (230) umfasst, der konfiguriert ist, um das Einführen einer oder mehrerer Vorrichtungen in den Saugkatheter (200) zu ermöglichen.

5. System nach Anspruch 4,
bei dem die ein oder mehreren Vorrichtungen eine Sonde (700) umfassen; oder
bei dem die eine oder die mehreren Vorrichtungen einen Steuerdraht (600) umfassen.

6. System nach Anspruch 4,
bei dem der mindestens eine Arbeitsanschluss (230) einen Tuohy-Borst-Adapter umfasst; oder
bei dem der mindestens eine Arbeitsanschluss (230) eine flüssigkeitsdichte Abdichtung um die ein oder mehreren Vorrichtungen herum bildet, wenn die ein oder mehreren Vorrichtungen in den Saugkatheter (200) eingeführt sind.

7. System nach Anspruch 4, bei dem der mindestens eine Arbeitsanschluss (230) durch einen Verbinder (240) lösbar mit der Steuereinheit (220) verbunden ist; und optional
bei dem der Verbinder eine Schnellkupplung ist.

8. System nach Anspruch 4, bei dem der mindestens eine Arbeitsanschluss (230) mindestens zwei Arbeitsanschlüsse (230, 232) mit unterschiedlichen Größen umfasst.

9. System nach Anspruch 1, bei dem der Saugkatheter (200) einen oder mehrere Steuerdrähte (218a, 218b) umfasst, die mit der steuerbaren Spitze verbunden sind, wobei die Steuereinheit (220) konfiguriert ist, um selektiv Spannung auf die ein oder mehreren Steuerdrähte (218a, 218b) auszuüben, um die steuerbare Spitze in die ein oder mehreren Richtungen zu biegen.

10. System nach Anspruch 1, wobei die steuerbare Spitze konfiguriert ist, um sich unter einem Winkel von etwa 0 bis etwa 90 Grad zu biegen.

11. System nach Anspruch 1, das ferner eine Filtervorrichtung (300) umfasst, die in Fluidverbindung zwischen dem Saugkatheter (200) und dem Rückführkatheter (500) positioniert ist, wobei die Filtervorrichtung (300) konfiguriert ist, um festes Material zurückzuhalten, das aus dem Körper des Patienten in den Saugkatheter (200) aufgenommen wurde.

12. System nach Anspruch 1, ferner umfassend einen Oxygenator (330), der konfiguriert ist, um dem Blut des Patienten Sauerstoff zuzuführen und/oder Kohlendioxid aus dem Blut des Patienten zu entfernen, wobei der Oxygenator (330) in Fluidverbindung zwischen dem Saugkatheter (200) und dem Rückführkatheter (500) positioniert ist.

13. System nach Anspruch 1, das ferner eine Sonde (700) umfasst, die bemessen und konfiguriert ist, um in einem Lumen des Saugkatheters (200) oder des Rückführkatheters (500) aufgenommen zu werden; und
optional bei dem die Sonde (700) eine steuerbare Spitze und eine Steuereinheit (220) umfasst, die von einem Benutzer betätigbar ist, um die steuerbare Spitze der Sonde in eine oder mehrere Richtungen zu biegen.

14. System nach Anspruch 1, wobei das System eine zweite Steuereinheit (520) umfasst und der Rückführkatheter (500) eine steuerbare Spitze (510) umfasst, die konfiguriert ist, um sich in Reaktion auf eine Betätigung der zweiten Steuereinheit (520) in eine oder mehrere Richtungen zu biegen.

## Revendications

1. Système (100) pour éliminer une matière indésirable à partir du corps d'un patient, le système comprenant : un cathéter d'aspiration (200) pouvant être inséré dans le patient comprenant une extrémité distale (210) présentant une pointe orientable configurée pour se plier dans une ou plusieurs directions, dans lequel l'extrémité distale (210) du cathéter d'aspiration (200) ne comprend ni un entonnoir ni une coupelle ; un dispositif de commande (220) couplé mécaniquement au cathéter d'aspiration (200) et pouvant être actionné par un utilisateur pour plier la pointe orientable dans les une ou plusieurs directions ; une pompe (400) comprenant un orifice d'entrée (410) et un orifice de décharge (420), l'orifice d'entrée (410) étant en communication fluidique avec le cathéter d'aspiration (200), la pompe (400) étant configurée pour générer une pression négative au niveau de l'orifice d'entrée (410) et une pression positive au niveau de l'orifice de décharge (420) en utilisation ; et un cathéter de retour (500) en communication fluidique avec l'orifice de décharge (420) de la pompe (400) et configuré pour être inséré dans le patient, dans lequel la matière indésirable comprend un ou plusieurs parmi des emboles, des thromboses, des tumeurs ou des débris.

2. Système selon la revendication 1,
dans lequel le cathéter d'aspiration (200) présente une taille égale ou supérieure à 10 Fr ; ou
dans lequel le cathéter d'aspiration (200) présente un diamètre de lumière d'au moins 3 mm ; ou
dans lequel la pression négative au niveau de l'orifice d'entrée (410) est comprise entre 0 mmHg et environ - 150 mmHg.

3. Système selon la revendication 1,
dans lequel la pompe (400) est configurée pour générer un débit d'écoulement d'au moins 100 mL/min à travers le cathéter d'aspiration (200) ; ou
dans lequel la pompe est configurée pour générer un débit d'écoulement d'environ 2 L/min à environ 4 L/min à travers le cathéter d'aspiration.

4. Système selon la revendication 1, comprenant en outre au moins un orifice de travail (230) configuré pour permettre l'insertion d'un ou de plusieurs dispositifs dans le cathéter d'aspiration (200).

5. Système selon la revendication 4,
dans lequel les un ou plusieurs dispositifs incluent un stylet (700) ; ou
dans lequel les un ou plusieurs dispositifs incluent un fil-guide (600).

6. Système selon la revendication 4,
dans lequel le au moins un orifice de travail (230) inclut un adaptateur Tuohy Borst ; ou
dans lequel le au moins un orifice de travail (230) fournit un joint étanche aux fluides autour des un ou plusieurs dispositifs lorsque les un ou plusieurs dispositifs sont insérés dans le cathéter d'aspiration (200).

7. Système selon la revendication 4, dans lequel le au moins un orifice de travail (230) est connecté de manière amovible au dispositif de commande (220) par un connecteur (240) ; et
facultativement dans lequel le connecteur est un raccord à connexion rapide.

8. Système selon la revendication 4, dans lequel le au moins un orifice de travail (230) comprend au moins deux orifices de travail (230, 232) présentant des tailles différentes.

9. Système selon la revendication 1, dans lequel le cathéter d'aspiration (200) inclut un ou plusieurs fils de direction (218a, 218b) connectés à la pointe orientable, le dispositif de commande (220) étant configuré pour appliquer sélectivement une tension sur les un ou plusieurs fils de direction (218a, 218b) afin de plier la pointe orientable dans les une ou plusieurs directions.

10. Système selon la revendication 1, dans lequel la pointe orientable est configurée pour se plier selon un angle d'environ 0 à environ 90 degrés.

11. Système selon la revendication 1, comprenant en outre un dispositif de filtre (300) positionné en communication fluidique entre le cathéter d'aspiration (200) et le cathéter de retour (500), le dispositif de filtre (300) étant configuré pour piéger une matière solide reçue dans le cathéter d'aspiration (200) à partir du corps du patient.

12. Système selon la revendication 1, comprenant en outre un oxygénateur (330) configuré pour ajouter de l'oxygène et/ou éliminer le dioxyde de carbone du sang provenant du patient, l'oxygénateur (330) étant positionné en communication fluidique entre le cathéter d'aspiration (200) et le cathéter de retour (500).

13. Système selon la revendication 1, comprenant en outre un stylet (700) dimensionné et configuré pour être reçu dans une lumière du cathéter d'aspiration (200) ou du cathéter de retour (500) ; et facultativement
dans lequel le stylet (700) comprend une pointe orientable et un dispositif de commande (220) pouvant être actionné par un utilisateur pour plier la pointe orientable du stylet dans une ou plusieurs directions.

14. Système selon la revendication 1, dans lequel le système inclut un second dispositif de commande (520) et le cathéter de retour (500) inclut une pointe orientable (510) configurée pour se plier dans une ou plusieurs directions en réponse au fonctionnement du second dispositif de commande (520).
